# EUROPEAN PATENT APPLICATION

(11) **EP 4 029 930 A1**
(43) Date of publication of application: **20.07.2022**
(21) Application number: 21152225.5
(22) Date of filing: 19.01.2021
(51) Int. Cl.: C12M 3/06, C12M 1/12, C12M 1/00, A61L 2/20

(54) **INCUBATOR SHAKER WITH OZONE DECONTAMINATION AND METHOD FOR DECONTAMINATING AN INCUBATOR SHAKER**

(71) Applicant: Damecx UG (haftungsbeschränkt), 75433 Maulbronn (DE)
(72) Inventor: Merk, Winfried, 76307 Karlsbad (DE)
(74) Representative: Gunzelmann, Rainer

(57) **Abstract**

Disclosed is an incubator shaker (1) for cell cultivation, which comprises an incubation chamber (10), a shaking table (21) disposed in the incubation chamber (10), and an ozone distributing device (100) for distributing ozone in the incubation chamber (10) including a space underneath the shaking table (21). The shaking table (21) is configured to place a cultivation container thereon during a cultivation process. This incubator shaker (1) can be used for a further disclosed method for decontaminating the incubator shaker (1), including the steps of placing the ozone distributing device (100) into the incubation chamber (10), guiding ozone into the incubation chamber (10), and conveying the ozone inside the incubation chamber (10) including a space underneath the shaking table (21).

## Description

The invention relates to an incubator shaker with ozone decontamination and a method for decontaminating an incubator shaker. Particularly, the invention relates to an incubator shaker including a shaking table and an ozone distributing device for an even ozone distribution throughout the entire incubation chamber, also underneath the shaking table, and a method for an automated decontamination of an incubator shaker including guiding ozone into a space underneath a shaking table.

Incubator shakers for cell cultivation, such as mammalian cells, include an incubation chamber that is usually kept under a specific atmosphere optimal for agitated cells to be cultivated. For instance, the temperature can be kept around 37°C with a relative humidity of approximately 80 to 95%, which prevents the culture medium (fluid) from extensive evaporation. Moreover, the carbon dioxide (COz or CO2) level of the atmosphere in the incubation chamber can be kept around approximately 5%, in order to keep a pH value of the culture medium constant. However, particularly the high relative humidity increases condensation inside the incubation chamber, and hence the risk of mould build-up.

Thus, the incubation chamber requires regular decontamination. Cleaning and disinfection is an integral part of a contamination control strategy in the pharmaceutical production following the guidelines of good manufacturing practice (GMP). Manual decontamination of all surfaces is usually achieved by wipe cleaning. For disinfection to be effective, cleaning to remove dirt, dust and soil from surfaces must be performed first. In addition, a disinfection agent should be employed and a sporicidal agent should periodically be used.

An automated decontamination with chemicals, e.g. hydrogen peroxide (H2O2), dry heat or moist heat is a common feature for incubators, used for static cell cultivation, i.e. without having an integrated (orbital) shaking mechanism. An automated decontamination in static CO2 incubators usually takes place by heating the interior of the incubation chamber for several hours, approximately above 120°C and preferably at approximately 160°C or 180°C. Another known efficient method for static incubators is using H2O2 vapour diluted to 6% concentration in example.

A way of decontamination is described in WO 2016/161155 A2, which comprises an incubator cabinet having a transfer chamber and an internal chamber, one or both of which can be filled with a sterilizing gas, such as ozone gas or hydrogen peroxide gas/vapor or both. WO 2005/060385 A2, likewise, describes a vapor decontamination system having a first generator for generating vaporized hydrogen peroxide to be introduced into a carrier gas and a device for introducing ozone into the carrier gas as well as a closed loop circulating system for supplying the vaporized hydrogen peroxide and carrier gas to a chamber.

However, hydrogen peroxide (H2O2) is decomposed to aqueous H2O and O2 gas, so that humidity will develop in the incubator and could enter the motor of the shaking drive. In addition, electronic and/or electric parts of a shaking mechanism inside the incubator may not be exposed to high temperatures.

It is therefore an object of the present invention to provide an incubator shaker that can be decontaminated in an efficient and easy manner, and further to provide an enhanced method for decontaminating an incubator shaker

This object is achieved by the invention in form of an incubator shaker with the features of claim 1, and a method with the features of claim 14.

According to an example to better understand the present disclosure, an incubator shaker for cell cultivation, e.g. in a suspension, comprises an incubation chamber, a shaking table disposed in the incubation chamber, and an ozone distributing device for distributing ozone in the incubation chamber including a space underneath the shaking table. The shaking table can be configured to place a cultivation container thereon.

The ozone distributing device provides for a uniform distribution and an overall even concentration of the ozone in the entire incubation chamber. Particularly, by providing a specified ozone concentration and even distribution underneath the shaking table an efficient decontamination in the entire incubation chamber can be achieved. A specified ozone concentration means that the ozone concentration is the same in each region of the incubation chamber.

Conventional decontamination processes in incubators require that interior components, such as racks and shelves for static cultivation have to be removed. A shaking table of an incubator shaker with built-in shaking drive motor however cannot be removed from the incubation chamber. If such interior components were not removed, the sterilizing gas may not reach areas covered by such interior components, particularly covered by a shaking table usually having a size almost equal to a bottom of the incubation chamber, thereby blocking the disinfecting gas. As a result, the disinfecting gas does not reach the space underneath the shaking table or only in such small concentration that it is not effectively decontaminating this region of the incubation chamber.

In accordance with the present disclosure, due to the distribution of the ozone in the entire incubation chamber an equal ozone concentration is achieved in the entire incubation chamber. Even in case of an incubator shaker with integrated rotary shaking drive motor, for example, a motor for orbital movement of the shaking table, an equal ozone concentration can be achieved throughout the entire incubation chamber including underneath the shaking table and around the motor. This further reduces the time for the decontamination process compared to conventional devices, since the process time does not depend on the smallest concentration of sterilizing gas in a particular region, i.e., a region not well reached by the sterilizing gas.

Ozone (O₃ or O3) is highly reactive with a very high oxidation potential, which stems from its ability to extract electrons from other molecules and release one of its own oxygen atoms in the process. Due to this powerful oxidizing property, ozone is very bactericidal, virucidal, sporicidal, and germicidal, much more than, for example, chloride or hydrogen peroxide. A good ozone concentration for decontamination of the incubation chamber is between 30 and 100 ppm, preferably 50 to 70 ppm and most preferably at approximately 60 ppm. Thus, in this disclosure any reference to conveying ozone or conveyed ozone means to convey a gas, such as air, with such ozone concentration. Conveying/conveyed ozone, hence, means that the ozone itself is distributed throughout the interior space of the incubation chamber and reaches all surfaces of the incubation chamber as well as interior components installed in the incubation chamber.

In an implementation variant the ozone distributing device can comprise a side panel having a plurality of openings and being arranged adjacent to a sidewall of the incubation chamber. While the incubation chamber sidewall delimits the incubation chamber at one side thereof, the side panel is located in front of the sidewall, i.e. inside of the incubation chamber.

The ozone distributing device can further comprise a conveying device configured to convey ozone into a first space between the side panel and a sidewall of the incubation chamber. The conveying device, hence, achieves a forced airflow carrying the ozone inside the incubation chamber. This forced flow of air and ozone facilitates providing every section and region of an interior space of the incubation chamber with ozone, even if complex interior structures are arranged inside the incubation chamber.

The conveyed ozone has to leave the first space, which is achieved, at least partially, by the plurality of openings in the side panel. Thus, the conveyed ozone streams through the first space and through the plurality of openings away from the sidewall of the incubation chamber, i.e. towards the centre of the incubation chamber. The arrangement of the side panel in the incubation chamber, hence, facilitates a uniform distribution of the ozone in the incubation chamber.

In a further implementation variant, at least one of the plurality of openings is arranged in the side panel at a height being below the shaking table. Since the side panel is arranged adjacent to the sidewall of the incubation chamber, i.e. arranged in a substantially vertical manner, and since the shaking table is usually arranged in a substantially horizontal manner inside of the incubation chamber, a portion of the interior space of the incubation chamber is underneath the shaking table and a corresponding section of the side panel can, thus, be arranged in this portion of the interior space of the incubation chamber. The at least one opening of the plurality of openings is arranged in this section of the side panel, so that ozone conveyed through the first space flows into the portion of the interior space of the incubation chamber underneath the shaking table. This facilitates an equal distribution of ozone throughout the entire interior space of the incubation chamber.

Additionally or alternatively, the lower edge of the side panel facing the bottom of the incubation chamber may include at least one recess. In other words, the side panel may have at least one foot portion standing on the bottom of the incubation chamber, while the lower edge of the side panel forms adjacent to the foot portion and elevated edge. This recess formed by the elevated edge has the same function as an opening in the side panel.

Also additionally or alternatively, the side panel may be mounted to the sidewall of the incubation chamber in such a manner that at least a portion of the lower edge does not contact the bottom of the incubation chamber, but is spaced apart therefrom.

In an optional variant, the plurality of openings in the side panel may be disposed from top to bottom of the side panel, so that at least some of the plurality of openings are also arranged in a section of the side panel corresponding to a portion of the interior space of the incubation chamber above the shaking table. The plurality of openings may be distributed in a row from top to bottom (substantially vertically), and may optionally also be distributed 2-dimensionally on the side panel (substantially vertically and horizontally distributed or distributed on a diagonal).

In another implementation variant, the ozone distributing device can further comprise a top panel having an opening and being arranged adjacent to a ceiling of the incubation chamber. In other words, like the side panel being arranged substantially vertically and substantially parallel to the sidewall of the incubation chamber, the top panel may be arranged substantially horizontally and substantially parallel to the ceiling of the incubation chamber.

Furthermore, the conveying device may be configured to convey ozone through the opening into a second space between the top panel and the ceiling of the incubation chamber. The conveying device, hence, conveys ozone from a central interior space of the incubation chamber into the section of the interior space of the incubation chamber arranged between the top panel at the ceiling of the incubation chamber, i.e. the second space.

In an optional variant, the conveying device may be arranged in the second space and conveys ozone through the opening in the top panel and into the second space. Alternatively or additionally, the conveying device may be arranged in the opening in the top panel.

The side panel can be coupled to the top panel, so that the first space is fluidly connected to the second space. In other words, a plane, in which the top panel is arranged, may form an angle with a plane, in which the side panel is arranged, wherein the angle is greater than 0° and smaller than 180°. The first and second space are delimited by the ceiling and sidewall of the incubation chamber in an outward direction and are delimited by the top panel and side panel in an inward direction (towards the centre of the incubation chamber). Thus, ozone conveyed by the conveying device streams through the opening in the top panel, through the second space, through the first space, and through the plurality of openings in the side panel. After this, the ozone may reach the conveying device again, so that a circular flow of ozone through the incubation chamber is achieved. This facilitates an equal distribution of the ozone in the entire incubation chamber, and speeds up the decontamination process.

In a further variant, the ozone distributing device may further comprise a conveying device arranged at a backside of the incubation chamber. The conveying device is configured to convey ozone through an interior space of the incubation chamber, particularly towards the centre of the incubation chamber.

For example, the ozone distributing device can further comprise a back panel arranged adjacent to the backwall of the incubation chamber. The back panel can form a third space between the back panel and a backwall of the incubation chamber. In other words, like the side panel being arranged substantially vertically and substantially parallel to the sidewall of the incubation chamber, the back panel may be arranged substantially vertically and substantially parallel to the backwall of the incubation chamber. The third space can be fluidly connected to the first space and/or second space.

Additionally or alternatively, the conveying device may be configured to convey ozone from the interior space of the incubation chamber (e.g., the centre of the incubation chamber) and into the third space behind the back panel. For example, the back panel may include an opening, through which the ozone is conveyed into the search space. Furthermore, the conveying device may be configured to convey the ozone through the search space towards the first space and/or the second space. Thus, the conveying device can then be operated in a manner similar to the one described with respect to the top panel.

Furthermore, the conveying device may be arranged outside of the incubation chamber, i.e. behind the backwall of the incubation chamber. It could be connected to a duct having an opening in the backwall, in order to suck in ozone from the interior of the incubation chamber. The conveying device may then convey the ozone into the incubation chamber through the backwall. Alternatively, conveying the ozone into the incubation chamber may be achieved through the same opening in the backwall or through a different opening.

In yet another implementation variant, the ozone distributing device can further comprise a filter arranged upstream of a conveying direction of the conveying device and configured to filter a gas before entering the conveying device. Such filter may be arranged at an inlet of the conveying device, so that a gas and particularly ozone is sucked into the conveying device through the filter. Likewise, the filter may be arranged in front of the opening in the top or the back panel, for example, covering the entire opening in the top or back panel. Thus, any gas and ozone conveyed by the conveying device and sucked through the opening and the top or back panel is filtered before entering the second space and the first space. This prevents contamination of the first space and second space as well as the conveying device. The filter, for example, can be a HEPA-filter (high-efficiency particulate air filter).

In another implementation variant, the conveying device can be a fan, such as a radial fan. Alternatively or additionally, the conveying device may be a venturi nozzle. Alternatively or additionally, the conveying device may include a pump. The conveying device may have an inlet, through which ozone enters the conveying device, and an outlet, through which the conveyed ozone leaves the conveying device towards the first and/or second space. For instance, the filter may be arranged in front of the inlet of the conveying device, while the outlet of the conveying device is disposed, for example, in front of or near the first space and/or the opening in the top panel.

In a further implementation variant, the conveying device can comprise a motor, which is arranged outside of the incubation chamber. For instance, the motor may have a shaft, that reaches through a wall of the incubation chamber (e.g. the ceiling, sidewall or backwall of the incubation chamber) into the interior space of the incubation chamber. A fan or blade may be arranged on this shaft constituting the conveying device. A gas-tight seal around the shaft separates electric components of the conveying device (i.e. the motor) from the interior space of the incubation chamber, so that ozone does not reach the electric components nor the ambient atmosphere around the incubator.

In another implementation variant, the incubator may further comprise a UV light source arranged in a region of the incubation chamber to which the conveying device conveys the ozone. This UV light source can be used after decontamination of the interior space of the incubation chamber, since UV light facilitates breakdown or decomposition of ozone. Thus, any remains of ozone after successful decontamination can be quickly removed from the interior space of the incubation chamber. The region, to which the conveying device conveys the ozone, may be the first space and/or the second space. Thus, the UV light source can be installed in this region.

The UV light source, arranged inside the incubation chamber, can further be used together with the conveying device during a cultivation process. By conveying the atmosphere inside the incubation chamber during the cultivation process and activating the UV light source, the conveyed atmosphere is decontaminated due to the disinfecting properties of UV light. The UV light should be arranged inside the incubation chamber in such a manner, that the UV light does not emit onto the cultivated cells, which may damage the cells. For instance, the top panel and the ceiling of the incubation chamber or the back panel and the backwall of the incubation chamber or the side panel and the sidewall of the incubation chamber may form a (bypass) channel for the conveyed atmosphere, wherein the top, back or side panel blocks any UV light from the UV light source to be admitted into the interior space of the incubation chamber.

In addition or alternatively, the incubator may further comprise a catalyst, for example a platinum catalyst, that is arranged in a region of the incubation chamber to which the conveying device conveys the ozone. Such catalyst can be configured to breakdown or decompose ozone, in order to facilitate removal of any remains of ozone from the interior space of the incubation chamber after the decontamination process. For instance, the catalyst and the UV light source may be installed as a structural unit in the region of the incubation chamber.

In a further implementation variant, the shaking table can comprise at least one opening. Such opening allows ozone to be conveyed from a space underneath the table towards the centre of the interior space of the incubation chamber, and further back to the conveying device. The distribution of ozone underneath the table can be improved, so that an efficient decontamination of all areas in the incubation chamber can be achieved. The at least one opening in the table can be sized and shaped to place a cultivation vessel onto the opening and holding the vessel in place, for example during a cultivation process. The cultivation vessel can be removed during the decontamination process. Alternatively or additionally, the at least one opening may be sized and shaped, in order to facilitate the flow of ozone underneath the table, in order to reach each section of the incubation chamber underneath the table. For instance, the at least one opening may be sized and shaped to optimize the flow/stream of ozone.

In yet another implementation variant, the incubator can further comprise a shaking device configured to move the shaking table. For instance, the shaking device may include a rotary drive motor for orbital movement. Thus, the shaking device together with the table may form an orbital shaking table. Such shaking table can be used for cultivation of cells that require mixing with the culture medium.

In a further implementation variant, the shaking device of the incubator shaker can comprise a shaft coupled to the table, wherein paddles or blades are connected to the shaft. The shaft may rotate or perform a rotational movement, in order to form an eccentric that allows (orbital) shaking of the table. This rotation or rotational movement can be used to further force the ozone underneath the table to move. For instance, the paddles connected to the shaft move together with the shaft. The movement of such paddles also moves the air and, hence, the ozone underneath the table.

The shaft may be driven by a motor arranged inside the incubation chamber. In this case, a housing is provided separating the motor, particularly its electric and electronic components, from the atmosphere inside the incubation chamber. This prevents humidity and ozone from reaching the motor during the cultivation process and the decontamination process, respectively. Such housing can be manufactured from or covered on the outside by a material that is easy to decontaminate, such as stainless steel. Optionally, the housing and motor may be installed in such a manner that they eccentrically move together with the table. In this case, the paddles or blades may be connected to the housing of the motor in addition or alternatively to the gas conveying a means connected to the shaft.

Alternatively, the motor may be arranged outside the incubation chamber. In this case, the shaft extends through the housing of the incubator or at least the bottom of the incubation chamber, which requires a fluid-tight seal (preventing gas and liquids from leaving the incubation chamber) around the shaft. In this case, the motor does not require a specific housing arranged inside the incubation chamber, which avoids a plurality of differently shaped surfaces in the space underneath the table and facilitates decontamination and cleaning the space underneath the table.

In yet a further implementation variant, the incubator can further comprise an inlet port configured to guide a gas into the incubation chamber. Such inlet port may simply be a duct, that has an outlet inside of the incubation chamber. Furthermore, the inlet port can be in fluid communication with an ozone source and a carbon dioxide source. For example, the inlet port may be connected to a duct being in fluid communication with the ozone source and may further be connected to a duct being in fluid communication with a carbon dioxide source (carbon dioxide is usually required during the cultivation process). In addition, optional valves may be employed in the respective duct, in order to allow ozone and carbon dioxide to be filled into the incubation chamber, respectively. Thus, the inlet port may be used to provide carbon dioxide into the incubation chamber, particularly during the cultivation process. Moreover, during decontamination the inlet port can further be used to provide ozone into the incubation chamber.

Alternatively or additionally, an ozone generator may be included in the incubator. For instance, the ozone generator may be arranged outside of the incubation chamber and could optionally be connected to the inlet port. According to another example, the ozone generator may be arranged inside of the incubation chamber. For example, the ozone generator may be arranged upstream or downstream of the conveying device, so that generated ozone is immediately distributed throughout the interior space of the incubation chamber. For instance, the ozone generator may be arranged in the first space and/or the second space and/or the third space "behind" the top panel and/or the side panel and/or back panel, respectively. Employing an ozone generator relieves from the burden of having an ozone tank or container, wherein pressurized ozone is stored.

In another implementation variant, the incubator can further comprise a door for closing an opening of the incubation chamber to the ambient environment, and a locking mechanism configured to lock the door in a closed position. The opening of the incubation chamber may be used to place culture vessels inside of the incubation chamber or take them out of the incubation chamber. Furthermore, the locking mechanism may lock the door during the decontamination process. Particularly, since ozone is a potential harmful gas, the incubation chamber can be sealed off (or can be made airtight), so that the interior space of the incubation chamber is sealed and locked, while ozone is present in the interior space of the incubation chamber.

The locking mechanism may further be connected to a sensor arranged inside the incubation chamber. Such sensor may measure an ozone concentration in the atmosphere inside of the incubation chamber. If the ozone concentration is above a threshold indicating a harmful ozone concentration, the locking mechanism blocks the door from being opened.

In accordance with another example to better understand the present invention, a method for decontaminating an incubator, according to the first example, can comprise the following steps.

The decontamination process starts optionally with a removal of interior components of the incubation chamber, particularly interior components that can be removed easily, such as trays, racks or the like. However, interior structures that are more difficult to remove, such as a fixed shaking mechanism with the shaking table can be left in the incubation chamber. After an appropriate manual cleaning technique removing all physical soiling of interior surfaces of the incubation chamber has been performed, the disinfection process can be started.

An ozone distributing device, for example according to the first example described above, is to be provided in the incubation chamber of the incubator shaker. Then, ozone can be guided into the incubation chamber or can be generated/produced in the incubation chamber, and the ozone is conveyed inside the incubation chamber, wherein conveying comprises guiding the ozone into a space underneath the shaking table. Conveying the ozone means to convey a gas, such as air, present inside of the incubation chamber that is supplemented with ozone. This conveying of ozone throughout the inside of the incubation chamber and particularly in a space underneath the shaking table achieves decontamination of the interior space of the incubation chamber. In more detail, interior surfaces of the incubation chamber and surfaces of interior components left in the incubation chamber come into contact with the ozone. Any contamination, such as bacteria cells, bacterial and fungal spores and other biological material that would be a contamination risk for the cell cultivation process, also comes into contact with the ozone. Due to the high reactivity of the ozone, such contamination is deactivated, i.e. the interior space of the incubation chamber is disinfected.

In an implementation variant, the decontamination process may be controlled by a timer, for example for approximately 3 hours, and/or a profile function, combining additional parameters such as temperature. Additionally or alternatively, test tubes or sensors may be placed into the incubation chamber, to test the efficiency of the ozone decontamination. For instance, the test tubes or sensors may include bioindicators, indicating an amount of biological material, which will be high in the beginning and decrease during decontamination. The decontamination process may then be controlled depending on signals derived from the test tubes or sensors, particularly when these signals indicate a sufficient decontamination, i.e. a deactivation to an acceptable kill rate level.

For example, the decontamination process parameters, such as number and duration of decontamination cycles at a specific ozone concentration, ozone concentration, exposure time and environmental conditions, can be automatically controlled by a control unit (e.g., a microprocessor).

In an implementation variant, the decontamination process can be controlled by an ozone sensor, which is placed inside the incubation chamber providing sensor data to the microprocessor control unit.

The decontamination process parameters, such as ozone concentration and exposure time, can be tested, optimized, recorded and stored based on a specific risk assessment criteria of the end user. The acceptable routine parameters can form an approved protocol, a Standard Operating Procedure (S.O.P.), which is the basis for validating a specific process, i.e. a production process under GMP rules. A fully automated decontamination process can be performed in fully reproducible quality minimizing the risk of human errors.

In a further implementation variant, the decontamination process may further include controlling a temperature inside of the incubation chamber, for example, between 30 to 60°C.

In another implementation variant, the method can comprise, before guiding ozone into the incubation chamber, locking a door of the incubation chamber, guiding carbon dioxide into the incubation chamber, and determining a carbon dioxide level in the incubation chamber after a predefined time span. This process allows checking whether the incubation chamber is sealed, if the door is closed and locked, or whether there is a leak.

In case of a leak, the carbon dioxide would escape from the incubation chamber. Thus, the method further comprises aborting the method, if the carbon dioxide level is below a threshold value. In case the carbon dioxide leaks from the incubation chamber, ozone would also leak. While ozone would be quite harmful to any person near the incubator, carbon dioxide is less harmful. Therefore, before starting the decontamination process, a test for leakages of the incubation chamber can be performed with carbon dioxide. Only if the incubation chamber passes the test, the decontamination process, i.e. the introduction and/or generation of ozone in the incubation chamber, is started.

Otherwise, the method of decontamination is aborted, i.e. the actual decontamination does not start. Additionally, an alarm (visually and/or audibly) may be raised, if the incubation chamber did not pass the leakage test with carbon dioxide.

The present disclosure is not restricted to the examples and variants in the described form and order. Specifically, the description of examples and implementation variants is not to be understood as a specific limiting grouping of features. It is to be understood that the present disclosure also covers combinations of the examples and variants not explicitly described. Thus, each variant or optional feature can be combined with any other example, variant, optional feature or even combinations thereof.

Preferred embodiments of the invention are now explained in greater detail with reference to the enclosed schematic drawings, in which
- Figure 1: schematically illustrates a front view or section through an incubator shaker;
- Figure 2: schematically illustrates a side view or cross-section through the incubator shaker;
- Figure 3: schematically illustrates a perspective view of a shaking table with openings for optimized ozone circulation;
- Figure 4: schematically illustrates a front view or section through a bottom portion of an incubator with a built-in shaking device; and
- Figure 5: schematically illustrates a flow diagram of a decontamination method.

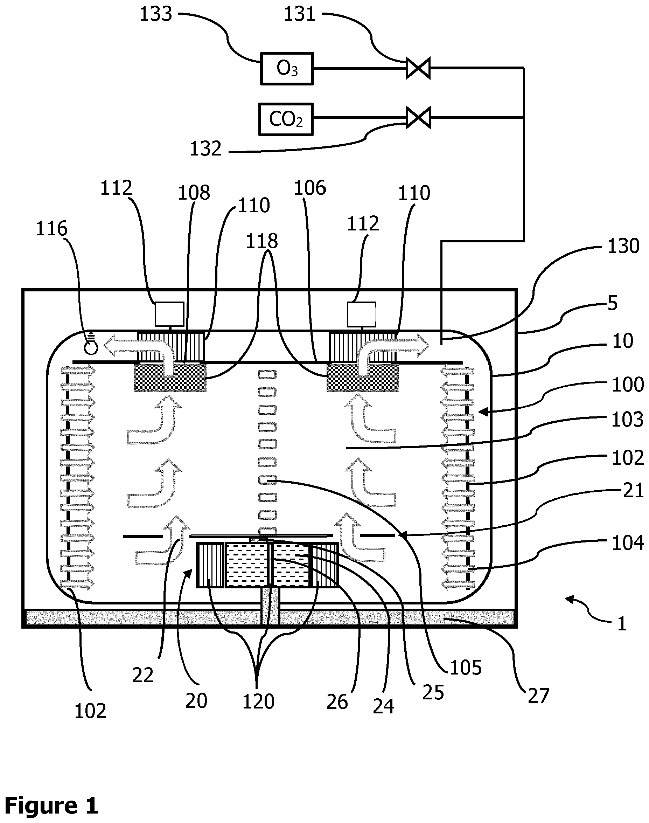

Figure 1 schematically illustrates a front view or section through an incubator shaker 1 for cell cultivation. The incubator shaker 1 includes an incubation chamber 10, which is defined by an outer housing 5 and an inner chamber 10. The inner chamber 10 is preferably made from stainless steel, wherein sidewalls, back wall, bottom and ceiling form a closed surface with rounded edges with a radius of 20 mm or more. This allows easy wipe cleaning of the surface of the inner chamber 10.

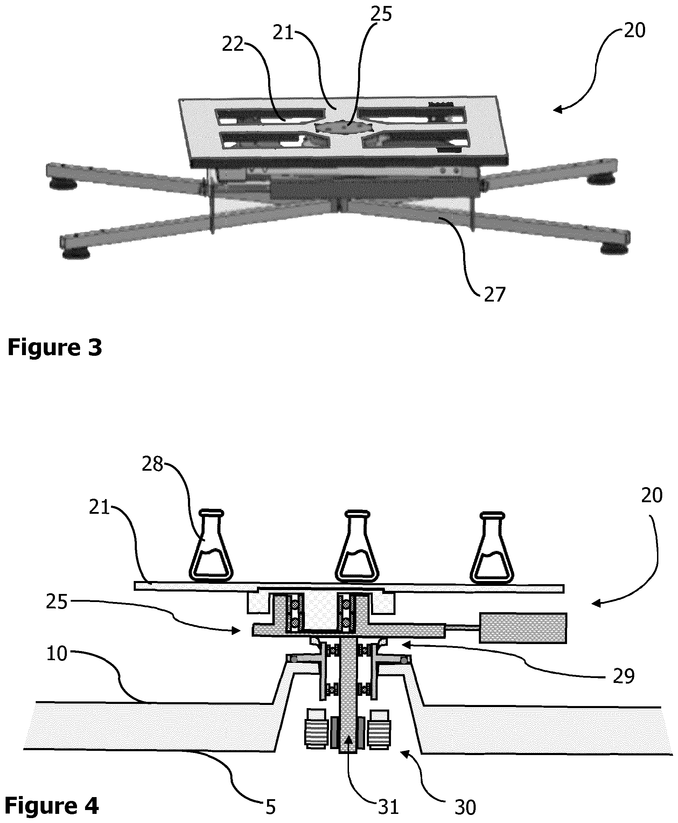

A shaking table 21 is placed inside the incubation chamber 10, which is separately illustrated in Figure 3. The shaking table 21 allows for placing a cultivation container 28 (Figure 4) thereon. For instance, a cultivation container 28 may be an Erlenmeyer flask or microtiter plate or a special cell culture flask, which contains a cell culture. The cultivation container 28 may directly be placed on the table 21, or alternatively, a tray (not illustrated) is placed on top of the shaking table 21, on which the cultivation container 28 is fixed.

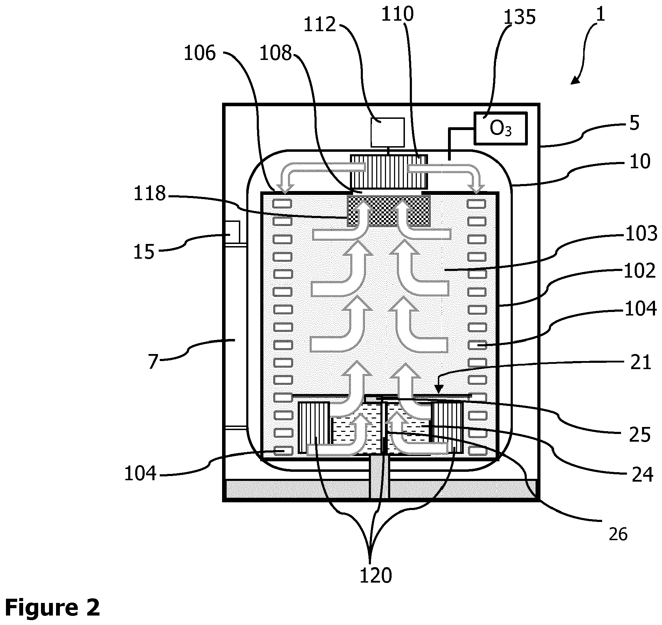

The incubator shaker 1 may further comprise an ozone distributing device 100 for distributing ozone in the incubation chamber 10, including distributing ozone underneath the shaking table 21. With reference to Figures 1 and 2, the latter of which schematically illustrates a side view or cross-section through the incubator shaker 1, the ozone distributing device 100 will be explained.

The most basic form of an ozone distributing device 100 is a side panel 102 having a plurality of openings 104 and being arranged adjacent to a sidewall of the incubation chamber 10. Two side panels 102 are illustrated in Figure 1 arranged adjacent to a respective sidewall of incubation chamber 10. In addition, a conveying device 110 conveys ozone into a first space between the side panel 102 and the sidewall of the incubation chamber. Although Figure 1 illustrates the conveying device 110 as being spaced apart from side panel 102, the conveying device 110 may be arranged inside of the incubation chamber 10 such that ozone conveyed by the conveying device 110 is directly conveyed into the first space. The conveyed ozone is then able to leave the first space via the openings 104 and towards the centre of the incubation chamber 10.

At least one of the plurality of openings 104 in the side panel 102 is arranged at a height being below the shaking table 21. As is illustrated with the plurality of arrows indicating the airflow through each of the plurality of openings 104 in Figure 1, at least a portion of the ozone streaming through the openings 104 is guided into the space of the incubation chamber 10 underneath the shaking table 21. Thus, efficient decontamination underneath the table is achieved by the ozone distributing device 100.

The ozone distributing device 100 may further comprise a top panel 106 arranged adjacent to the ceiling of the incubation chamber 10. The top panel 106 may also have an opening 108. The conveying device 110 can be configured to convey ozone through the opening 108 in the top panel 106 into a second space between the top panel 106 and the ceiling of the incubation chamber 10. For example, the conveying device 110 may transport ozone (e.g. air with a predefined ozone concentration) from an interior space of the incubation chamber 10 into the second space. The side panel 102 can be coupled to the top panel 106 in such a manner, that the first space is fluidly connected to the second space. Thus, any gas/ozone conveyed by the conveying device 110 into the second space has to stream towards the first space and the openings 104 in the side panel 102.

A filter 118 can be arranged upstream of the conveying device 110, in order to filter any particles from the ozone before entering the conveying device 110, the second space and the first space. As can be derived from the arrows depicted in Figures 1 and 2, a forced airflow is achieved through the incubation chamber 10. In order to increase efficiency of the decontamination, the incubator shaker 1 may include more than one conveying device 110, two of which are illustrated in Figure 1. In combination with a second side panel 102, a symmetric forced airflow, and hence ozone flow, can be achieved for efficient decontamination.

In addition to the side panel(s) 102, a back panel 103 may further be arranged adjacent to a back wall of the incubation chamber 10, thereby forming a third space between the back panel 103 and the back wall of the incubation chamber 10. As with the side panel(s) 102, ozone may be conveyed by the conveying device 110 from the second space into the third space, from which the ozone may enter the interior space of the incubation chamber 10 again via corresponding openings 105 provided in the back panel 103.

In an optional configuration the back panel 103 may also have an opening (not illustrated), such as opening 108 in top panel 106. The or a conveying device 110 can be configured to convey ozone through the opening in the back panel 103 into a third space between the back panel 103 and the back wall of the incubation chamber 10. For example, the conveying device 110 may transport ozone (e.g. air with a predefined ozone concentration) from an interior space of the incubation chamber 10 into the third space. The side panel 102 can be coupled to the back panel 103 in such a manner, that the first space is fluidly connected to the third space. Thus, any gas/ozone conveyed by the conveying device 110 into the third space has to stream towards the first space and the openings 104 in the side panel 102.

Likewise, a filter (not illustrated), such as filter 118 at top panel 106, can be arranged upstream of the conveying device 110 arranged at the back panel 103, in order to filter any particles from the ozone before entering the conveying device 110, the third space and the first space. As can be derived from the arrows depicted in Figures 1 and 2, a forced airflow is achieved through the incubation chamber 10. In order to increase efficiency of the decontamination, the incubator shaker 1 may include more than one conveying device 110, preferably two, left and right at the back panel. In combination with a second side panel 102, a symmetric forced airflow, and hence ozone flow, can be achieved for efficient decontamination.

The shaking table 21 may comprise at least one opening 22 (Figures 1 and 3), which allows a flow of ozone from the space underneath the shaking table 21 towards the centre of the incubation chamber 10, and hence towards the conveying device 110. Thus, the space underneath the shaking table 21 can be ventilated with the optimal ozone concentration as in the remaining regions of the incubation chamber 10, which allows an efficient decontamination underneath the shaking table 21.

A motor 112 of the conveying device 110 may be arranged outside of the incubation chamber 10, for example, in a space between outer housing 5 and incubation chamber 10. This prevents any electric component from coming into contact with the ozone during decontamination. Alternatively, the motor 112 may also be arranged outside housing 5.

The incubator shaker 1 may further comprise a shaking device 20 configured to shake the table 21. Exemplary shaking devices 20 are illustrated in Figures 3 (mobile shaking device) and Figure 4 (built-in shaking device), while another exemplary installation is illustrated in Figures 1 and 2. For instance, the shaking table 21 may be installed on an eccentric 25 connected to a motor driven rotor 24, so that the table 21 can be moved in an orbit or circle. This allows shaking of any flask 28 arranged on the table 21.

This rotational movement can further be used to convey the ozone in the space underneath the table 21. For example, the motor driven rotor 24 may be equipped with at least one gas conveying means 120, such as a paddle or blade. This gas conveying means 120 moves together with the motor driven rotor 24 and, hence, moves the ozone in the space underneath the shaking table 21, so that a good ventilation is achieved, and the ozone flows substantially equally through every section of the space underneath the shaking table 21.

The motor driven rotor 24 is turning around a fixed shaft 26 which functions as a stator as illustrated in Figures 1 and 2. This exemplary arrangement of the shaking device 20 inside of the incubation chamber 10 allows having a flat bottom wall of the incubation chamber 10. Thus, the bottom wall of the incubation chamber 10 can easily be cleaned. The space underneath the shaking device 20 can still be efficiently decontaminated due to the good ventilation of ozone underneath the table 21 and underneath the shaking device 20. The motor driven rotor 24 may have a function of a housing around the motor (not separately illustrated) separating the motor, particularly its electric and electronic components, from the atmosphere inside the incubation chamber 10. Such housing, for example made from easy-to-clean stainless steel, prevents humidity and ozone from reaching the motor. The gas conveying means 120 can then additionally or alternatively be mounted on the housing, particularly when the housing is turning around the shaft 26 with high speed during the decontamination process. An optional stand 27 of a mobile shaking device 20 (Figure 3) can be placed outside of the incubation chamber 10 during the decontamination process or may be placed onto the bottom wall of the incubation chamber 10 (not illustrated).

Alternatively, as illustrated in Figure 4 the bottom wall of the incubation chamber 10 may include a protrusion covering the motor 30, so that the motor 30 is outside of the incubation chamber 10. This allows the motor 30 to be installed without a specific fluid-tight housing. The rotating shaft 31 should at least include a portion extending through the incubation chamber 10 or through the incubation chamber 10 and housing 5 (as illustrated in Figure 4). In any case, a fluid-tight seal 29 is to be employed to seal the rotating shaft 31 connecting the motor 30 and the table 21. Although the surface of the bottom wall of the incubation chamber 10 has a more complex structure and/or sealing elements are required at the rotating shaft 31 and/or shaking table 21, decontamination is still efficient due to the good ventilation and provision of ozone underneath the shaking table 21. Moreover, a stand 27 for the shaking device 20 (as in Figure 3) may not be required, since the protrusion of the bottom wall of the incubation chamber 10 can be formed to provide sufficient structural stability for the shaking device 20.

The incubator 1 may further comprise an inlet port 130 configured to guide a gas into the incubation chamber 10. This inlet port 130 may be in fluid communication with an ozone source 131, illustrated as a valve. In addition thereto, the inlet port 130 may be in fluid communication with a carbon dioxide source 132, also illustrated as a valve. Thus, the inlet port 130 required for the carbon dioxide introduction for the cultivation process may also be used for the introduction of ozone during the decontamination process. Therefore, no additional inlet port and associated sealing is required.

The ozone may be produced in an ozone generator 133. In Figure 1, the ozone generator 133 is illustrated as a device being located outside of the incubator 1. For instance, the ozone generator 133 may be in fluid communication with the inlet port via valve 131. Alternatively, an ozone source in form of a pressure storage may be used instead of the ozone generator 133.

Alternatively or additionally, as is illustrated in Figure 2, an ozone generator 135 may be included in the incubator 1. Thus, no additional storage for ozone is required and the ozone may be generated directly from oxygen present in the air and atmosphere in and around the incubator 1.

As a further exemplary variant, the ozone generator 135 may be arranged inside of the incubation chamber 10 (instead of the position illustrated in Figure 2 between incubation chamber 10 and outer housing 5). This would even allow avoiding an inlet port for ozone through the incubation chamber 10. For example, ozone generator 135 may be arranged in the second space, i.e. between the top panel 106 and the ceiling of the incubation chamber 10, and/or in the first or third space, i.e. between the side panel 102 and a sidewall of the incubation chamber 10 or the back panel 103 and a back wall of the incubation chamber 10, respectively.

The interior space of the incubation chamber 10 may be reached through a door 7 closing an opening of the incubation chamber 10 to the ambient environment. In order to prevent ozone from exiting the incubation chamber 10 during the decontamination process, the door 7 may be locked by a locking mechanism 15. This locking mechanism 15 may be under control of one or more sensors, that measure an ozone concentration inside of the incubation chamber 10.

In order to decompose ozone after the decontamination process, and in order to release the locking mechanism 15, so that the door 7 can be opened again, a UV light source 116 can be installed inside of the incubator 1. Particularly, the UV light source 116 can be arranged inside of the incubation chamber 10. Preferably, the ozone conveyed by conveying device 110 passes the UV light source 116, so that the ozone is decomposed while being conveyed through the incubation chamber 10.

In addition or alternatively, a catalyst (not illustrated) can be arranged in a similar manner inside the incubator 1 or inside of the incubation chamber 10. If the catalyst is a passive catalyst, it should be sealed from the conveyed ozone during the decontamination process and should be open for the ozone to pass over/through the catalyst after the decontamination process, in order to decompose the ozone. For instance, a bypass (not illustrated) may be provided in the incubator 1, which can be sealed off from the incubation chamber 10 (via valves or doors) and which may be open to the flow of ozone induced by the conveying device 110 for decomposition of ozone.

The ozone distributing device 100 may be designed to be at least partially removable from the incubation chamber 10. This allows wipe cleaning of the incubation chamber 10, for example, before a disinfection process using ozone is started. Moreover, at least a part of the ozone distributing device 100 could be removable. In order to reach the UV light source or the ozone generator, both items may require replacement after a certain number of decontamination processes.

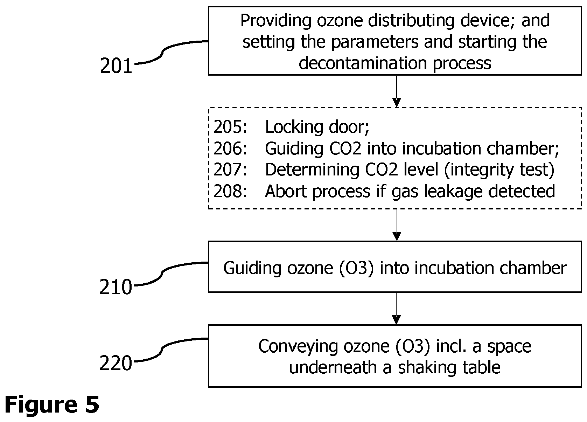

Figure 5 schematically illustrates a flow diagram of a decontamination method using the incubator shaker 1. In a first step 201, the ozone distributing devices provided, for example, provided to an incubator. The decontamination process is then started. For example, process parameters may be set and confirmed by a user. Thereafter, the ozone distributing device 110 is capable of providing ozone in the incubation chamber 10 and to produce a forced airflow through the incubation chamber 10, wherein the air can comprise ozone. This provision and conveying of ozone may be fully automated, particularly when process parameters are set upfront.

In step 210 ozone is guided into the incubation chamber 10. This may be achieved by opening a valve 131 fluidly connected to an ozone source 133 and an inlet port 130 opening into the interior space of the incubation chamber 10. Alternatively, an ozone generator 135 may be operated to generate ozone directly inside of the incubator 1 and/or inside of the incubation chamber 10.

Afterwards, in step 220, the ozone (enriched air) is conveyed throughout the interior space of the incubation chamber 10. This conveying includes distributing ozone even in a space under a shaking table 21 arranged in the incubation chamber 10. During step 220, further ozone may still be guided into or be produced in the incubation chamber 10 (step 210).

After a predefined time span, the decontamination process is stopped, i.e. the guiding or generation of ozone in the incubation chamber 10 (step 210) is stopped. The conveying of the ozone and air may be continued (step 220) as long as the ozone is decomposed. This decomposition of the ozone may be accelerated by switching on a UV light source and/or by guiding the ozone through a catalyst.

In order to protect the ambient environment around the incubator 1 from the harmful ozone, a test may be run before guiding the ozone into the incubation chamber 10 (step 210). Particularly, the optional steps (see dashed-lined square in Figure 5) may represent such testing. Firstly, in step 205, the door 7 of the incubation chamber 10 is locked, and in step 206, carbon dioxide is guided into the incubation chamber 10.

In step 207, a carbon dioxide level is determined inside the incubation chamber 10 and compared to a threshold. If the carbon dioxide level is above the threshold, it is very likely that the incubation chamber 10 is airtight, i.e. does not leak a gas, such as carbon dioxide or ozone. If the carbon dioxide level, however, is below the threshold, the decontamination process is aborted in step 208, i.e. steps 210 and 220 are not performed, since the low carbon dioxide level is an indication for a leakage.

## Claims

1. Incubator shaker (1) for cell cultivation, comprising:
an incubation chamber (10);
a shaking table (21) disposed in the incubation chamber (10), wherein the shaking table (21) is configured to place a cultivation container thereon; and
an ozone distributing device (100) for distributing ozone in the incubation chamber (10) including a space underneath the shaking table (21).

2. Incubator shaker (1) according to claim 1, wherein the ozone distributing device (100) comprises:
a side panel (102) having a plurality of openings (104) and being arranged adjacent to a sidewall of the incubation chamber (10); and
a conveying device (110) configured to convey ozone into a first space between the side panel (102) and the sidewall of the incubation chamber (10).

3. Incubator shaker (1) according to claim 2, wherein at least one of the plurality of openings (104) is arranged in the side panel (102) at a height being below the shaking table (21).

4. Incubator shaker (1) according to claim 2 or 3, wherein the ozone distributing device (100) further comprises:
a top panel (106) having an opening (108) and being arranged adjacent to a ceiling of the incubation chamber (10),
wherein the conveying device (110) is configured to convey ozone through the opening (108) into a second space between the top panel (106) and the ceiling of the incubation chamber (10), and
wherein the side panel (102) is coupled to the top panel (106), so that the first space is fluidly connected to the second space.

5. Incubator shaker (1) according to one of claims 2 to 4, wherein the ozone distributing device (100) further comprises:
a filter (118) arranged upstream of a conveying direction of the conveying device (110) and configured to filter a gas before entering the conveying device (110).

6. Incubator shaker (1) according to one of claims 2 to 5, wherein the conveying device (110) comprises a motor (112), wherein the motor (112) is arranged outside of the incubation chamber (10).

7. Incubator shaker (1) according to one of claims 2 to 6, further comprising:
a UV light source (116) arranged in a region of the incubation chamber (10) to which the conveying device (110) conveys the ozone.

8. Incubator shaker (1) according to one of claims 1 to 7, wherein the table (21) comprises at least one opening (22).

9. Incubator shaker (1) according to one of claims 1 to 8, further comprising:
a shaking device (20) configured to move the shaking table (21).

10. Incubator shaker (1) according to claim 9, wherein the shaking device (20) comprises an eccentric (25) coupled to the shaking table (21), and wherein paddles (120) are connected to the eccentric (25)

11. Incubator shaker (1) according to one of claims 1 to 10, further comprising:
an inlet port (130) configured to guide a gas into the incubation chamber (10),
wherein the inlet port (130) is in fluid communication with an ozone source (131) and a carbon dioxide source (132).

12. Incubator shaker (1) according to one of claims 1 to 11, further comprising:
an ozone generator (133) arranged inside the incubation chamber.

13. Incubator shaker (1) according to one of claims 1 to 12, further comprising:
a door (7) closing an opening of the incubation chamber (10) to the ambient environment; and
a locking mechanism (15) configured to lock the door (7) in a closed position.

14. Method for decontaminating an incubator shaker (1), according to one of claims 1 to 13, the method comprising:
providing (201) the ozone distributing device (100);
guiding (210) ozone into the incubation chamber (10); and
conveying (220) the ozone inside the incubation chamber (10), wherein conveying comprises guiding the ozone into a space underneath the table (21).

15. Method according to claim 14, wherein the method comprises, before guiding (210) ozone into the incubation chamber (10):
locking (205) a door of the incubation chamber (10);
guiding (206) carbon dioxide into the incubation chamber (10);
determining (207) a carbon dioxide level in the incubation chamber (10) after a predefined time span; and
if the carbon dioxide level is below a threshold value, aborting (208) the method.
